(19) Europäisches Patentamt
European Patent Office
Office européen des brevets

(11) EP 3 466 947 A1

(12) # EUROPEAN PATENT APPLICATION
published in accordance with Art. 153(4) EPC

(43) Date of publication:
**10.04.2019 Bulletin 2019/15**

(21) Application number: **17745766.0**

(22) Date of filing: **24.05.2017**

(51) Int Cl.:
***C07D 471/04*** (2006.01) ***A23G 1/48*** (2006.01)
***A23G 1/50*** (2006.01) ***A23G 1/54*** (2006.01)
***A23P 10/10*** (2016.01) ***A23P 20/10*** (2016.01)
***A23G 3/56*** (2006.01) ***A23L 19/00*** (2016.01)

(86) International application number:
**PCT/ES2017/070351**

(87) International publication number:
**WO 2017/203086 (30.11.2017 Gazette 2017/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.05.2016 ES 201630666**

(71) Applicants:
- **Consejo Superior De Investigaciones Científicas 28006 Madrid (ES)**
- **Katholieke Universiteit Leuven 3000 Leuven (BE)**

(72) Inventors:
- **GARCÍA FRUTOS, Eva María 28006 Madrid (ES)**
- **MARTÍN ÁLVAREZ, Cristina 3000 Leuven (BE)**
- **KENNES, Koen 3000 Leuven (BE)**

(74) Representative: **Pons Ariño, Angel**
**Pons Patentes y Marcas Internacional, S.L.**
**Glorieta Rubén Dario 4**
**28010 Madrid (ES)**

(54) **SYSTEMS BASED ON 5-BROMO-7-AZAINDOLE DERIVATIVES AS FUTURE SOLID EMITTERS**

(57) The present invention relates to a family of compounds derived from 5-bromo-7-azaindole to which alkyl chains have been incorporated in one of the nitrogens to make them soluble in organic solvents as well as the incorporation in the position 5 of 4-(diphenylamino)phenylboronic acid derivatives. These structural modifications increase the emission of the derivative in a solid state. The incorporation of aldehyde and cyanoacetic acid derivatives in position 3 facilitates the attachment thereof in different systems. Given this characteristic, they may be applicable in the development of solid-state luminescent materials used in applications of detection, biomedical applications, solid-state illumination, as well as optoelectronic devices.

EP 3 466 947 A1

## Description

The present invention relates to a family of compounds derived from 5-bromo-7-azaindole to which alkyl chains have been incorporated in one of the nitrogens to make them soluble in organic solvents. The incorporation in position 5 of 4-(diphenylamino)phenylboronic acid derivatives increases the emission of the solid-state derivative due to the lack of rotation of said nucleus. The incorporation of aldehyde and 2-cyano-but-2-enoic acid derivatives in position 3 facilitates the attachment thereof in different systems. Given this characteristic, they may be applicable in the development of solid-state luminescent materials used in detection, biomedical, solid state illumination applications, as well as optoelectronic devices.

## STATE OF THE ART

The study of new organic luminescent materials has been widely developed over the last few decades (Chem. Soc. Rev., 2012, 41, 211). Although the initial fundamental study of these luminescent organic materials is done in solution, their real application is usually in a solid state (J. Mater. Chem. C, 2014, 2, 3756). The synthesis of luminescent organic materials with a high degree of solid-state light emission is one of the most developed fields in recent years. The aggregation of the different chromophores has two effects on the luminescence processes. The two phenomena are completely different. One of these phenomena known as "aggregation-caused quenching" (ACQ), produces a reduction in the emission, a destructive effect for practical applications, a phenomenon in which the majority of materials which are fluorescent in solution. The other phenomenon is known as "Aggregation-induced emission" (AIE) (Chem. Soc. Rev. 2014, 43, 4494) or "aggregation-induced emission enhancement" (AIEE). In this case, potentially luminescent materials are never or rarely emitters in very diluted solutions, but they become highly emissive in concentrated solutions. These aspects are very important for solid-state applications. The reasons for this phenomenon are due to restrictions to intramolecular rotation (IMR), the formation of aggregates, a stronger flatness, the suppression of twisted intramolecular charge transfer (TICT) or the existence of excited state intramolecular proton transfer (ESIPT). An enormous variety of AIE molecules have been developed (Chem. Rev. 2015, 115, 11718), with great structural diversity: classic AIE luminogens, such as siloles, tetraphenylethene (TPE) and cyano-stilbene derivatives, or others which are not classic, such as derivatives of 1, 3, 4 oxadiazole, carbazoles and dendritic systems (Soft Matter 2013, 9, 4564).

Document PCT/ES2016/070094 describes a series of 7-azaindole derivatives capable of self-assembly to form organogels and xerogels with significant fluorescent properties.

Document ES201530966 describes a compound derived from 7,7'-diaza isoindigo, soluble in polar organic solvents which, by generating an organogel, have an induced emission in the red region of the visible spectrum (600-800 nm) and which may applicable to optoelectronic devices or fluorescent sensors.

## DESCRIPTION OF THE INVENTION

The inventors of the present invention have developed fluorescent systems based on 5-bromo-7-azaindole derivatives as future solid emitters. Furthermore, incorporating 2-cyano-but-2-enoic acid in position 3 of the azaindole gives rise to what is known as "aggregation-induced emission" (AIE). This implies that the compound has a high degree of light emission in a solid state or aggregation. Given this characteristic, the compounds of the invention may be applicable in the development of luminescent or electroluminescent materials in a solid state for detection, biomedical and solid-state lighting applications, as well as for optoelectronic devices, such as organic light-emitting diodes (OLEDs).

In a first aspect, the present invention relates to a compound of formula (I):

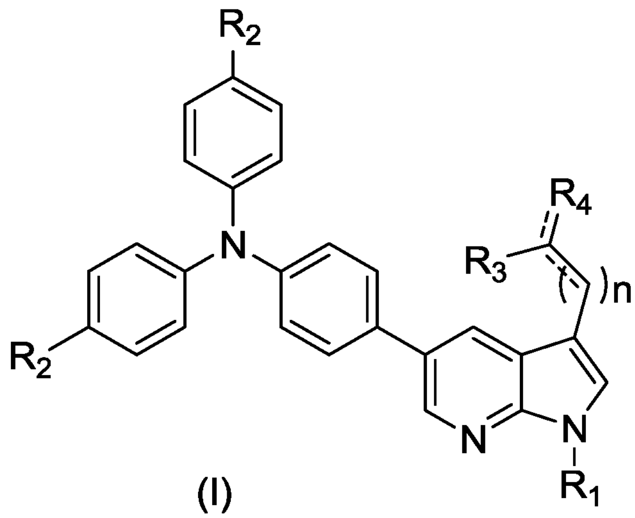

(I)

wherein $R_1$ is a $C_1$-$C_{20}$ alkyl; $R_2$ is selected from H, $C_1$-$C_6$ alkyl or O-$C_1$-$C_6$ alkyl; $R_3$ is selected from H, CN or CHO; $R_4$ is selected from COOH, CN, O or $CONH_2$; n is selected from 0 or 1.
and wherein - - - - represents a bond which may be double or single depending on the rest of the carbon atom bonds it is made up of.

In the present invention, the term "alkyl" relates to aliphatic, linear or branched chains, which have 1 to 20 carbon atoms, for example methyl, ethyl, *n*-propyl, *i*-propyl, *n*-butyl, *terc*-butyl, *sec*-butyl, pentyl or dodecyl, etc. Preferably the alkyl group has 4 to 8 and 10 to 18 carbon atoms. Optionally the alkyl groups may be substituted by one or more substituents, such as halogen, hydroxyl, azide, carboxylic acid or a substituted or non-substituted group, selected from among amino, amido, carboxylic ester, ether, thiol, acylamino or carboxamide.

In a preferred embodiment, $R_1$ is a $C_4$ to $C_8$ alkyl.

In another preferred embodiment, $R_1$ is a $C_{10}$ to $C_{18}$ alkyl. In a more preferred embodiment, $R_1$ is a $C_{12}$ alkyl .

In another preferred embodiment, $R_2$ is H.

In another preferred embodiment, n is 0.

In another preferred embodiment, $R_3$ is H.

In another preferred embodiment, $R_4$ is O.

In a more preferred embodiment, the compound of formula (I) has the following formula:

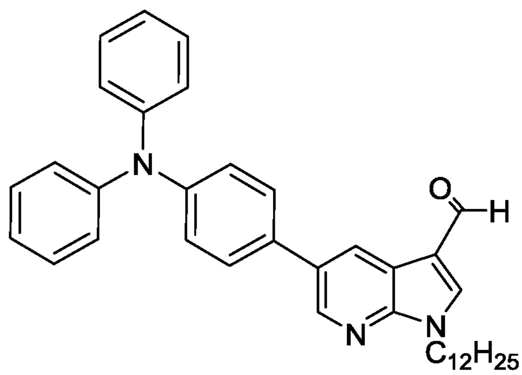

In another preferred embodiment, n is 1.

In another preferred embodiment, $R_3$ is COOH.

In another preferred embodiment, $R_4$ is CN.

In another more preferred embodiment, the compound of formula (I) has the following formula:

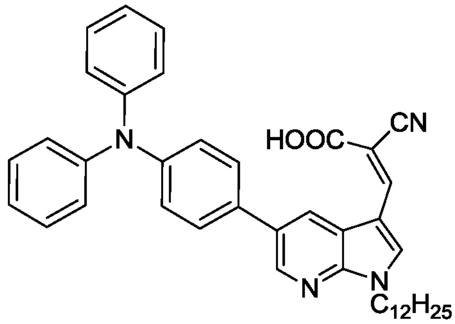

Another aspect of the invention relates to a luminescent material comprising the compound of formula (I), as described above.

Another aspect of the invention relates to the use of the compound of formula (I) for the manufacturing of luminescent or electroluminescent materials, which may be found in solid or liquid state and which may also be in aggregation in both states.

The term "aggregations" or "aggregate" in the present invention refers to the set of molecules of the compound of the invention interacting with each other, thereby having the AIE effect.

Another aspect of the invention relates to a device which comprises at least the luminescent or electroluminescent material, which may be found in solid or liquid state and which may also be in aggregation in both states, as previously described.

In another preferred embodiment, the device is an optoelectronic device, more preferably an organic light-emitting diode (OLED) device. These devices contain structural elements known by any person skilled in the art as an anode, a cathode and an active material. In a preferred embodiment, the device comprises a layer of active material which is the electroluminescent material of the invention and, optionally, an electron injection layer which improves electron-hole recombination. In a more preferred embodiment, the electron injection layer is made up of 2-(4-*tert*-butylphenyl)-5-(4-butylphenyl)-1,3,4-oxadiazole (PBD).

Throughout the description and the claims, the word "comprises" and its variants are not intended to exclude other technical characteristics, additives, components or steps. For those skilled in the art, other objects, advantages and characteristics of the invention may be deduced from both the description and the practical use of the invention. The following examples and drawings are provided by way of illustration, and are not meant to limit the present invention.

## BRIEF DESCRIPTION OF THE DRAWINGS

FIG. 1. Shows the absorption spectra of compounds **3** and **4** in dichloromethane in different concentrations.

FIG. 2. Shows the emission spectra **3** ($\lambda_{exc}$ =352 nm) and **4** ($\lambda_{exc}$= 391 nm) in dichloromethane and concentrations of $10^{-5}$ M.

FIG. 3. Shows the emission of compounds **3** (left) and **4** (right) in dichloromethane in a concentration of $10^{-6}$ M with the $\lambda$=365 nm lamp.

FIG. 4. Shows the absorption spectrum of the derivative **4** on film deposited by means of drop-casting on dichloromethane on the quartz slide.

FIG. 5. Shows the emission spectrum of **4** in solid state ($\lambda_{exc}$ .=391 nm).

FIG. 6. Shows the emission of compounds **3** (left) and **4** (right) in their solid state exited with a $\lambda$=365 nm lamp.

FIG. 7. Shows the absorption spectra of compound **4** in THF and in different proportions of THF mixture: water in a concentration of $10^{-6}$ M.

FIG. 8. Shows the emission of compound **4** in THF and in different proportions of THF: water in a concentration of $10^{-6}$ M with the lamp of $\lambda$=365 nm.

FIG. 9. Shows the emission spectra of compound **4** in THF and in different proportions of THF mixture: water in a concentration of $10^{-6}$ M.

FIG. 10. **A**) and **C**) show the intensity curve versus voltage and **B**) and **D**) show the electroluminescence spectra of compound **3** using two different architectures for making the device (device A and B, respectively). The figures inserted in figures A and C provide diagrams of each configuration of the device used in each case, as well as the energy diagram of the materials required for the embodiment thereof.

FIG. 11. Shows a comparison for the derivative **3** of the normalized electroluminescence spectra at different voltages applied for two different configurations of optoelectronic devices, where **A**) is device A and **B**) is device B.

FIG. 12. **A**) Shows a comparison for the derivative 3 of the deconvolution obtained from the electroluminescence spectra acquired from the configuration of the optoelectronic system B (device B). **B**) Shows the dependence of each component versus the voltage applied in each case.

FIG. 13. **A)** Shows the current - voltage curve and **B)** shows the electroluminescence spectra of compound **4** for the optoelectronic device designed for compound **4.** The figures inserted in figures 13A provide diagrams of the configuration of the device, as well as the energy diagram of the materials required for the embodiment thereof.

FIG. 14. Shows a comparison of electroluminescence spectra (obtained at different voltages) of the device made with compound **4.**

FIG. 15. Shows a study on the stability over time of the device prepared for compound 4, comparing **A)** current - voltage and **B)** the electroluminescence spectra of two optoelectronic devices, just after their preparation and 5 weeks after their preparation. It is worth mentioning that during this time, the device was stored exposed to the air with no type of seal.

## EXAMPLES

The invention is illustrated below by means of tests which reveal the effectiveness of the product of the invention.

### Example 1: synthesis of the compounds of formula (I) of the invention Synthesis of 5-bromo-1-dodecane-1*H*-pyrrole[2,3-*b*]pyridine (1)

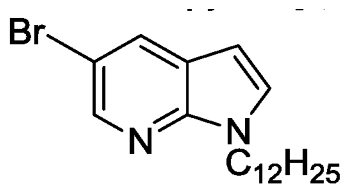

In a flask, 5-bromo-7-azaindole (350 mg, 1.786 mmol), KOH (152 mg, 2.715 mmol), tetrabutylammonium hydrogensulfate (32 mg, 0.09 mmol), and 1-iododecane (0.6 ml, 2.447 mmol) were mixed in 20 mL of acetone. The reaction was heated at 75°C for 24 hours. The mixture was cooled at room temperature. The solvent was eliminated at low pressure and water was added. The organic phase was extracted with $CH_2Cl_2$, dried with anhydrous $MgSO_4$ and filtered. The dichloromethane was eliminated at low pressure and the solvent was left to evaporate. The product was purified in a chromatographic column using silica gel (hexane; 1:1 hexane: $CH_2Cl_2$). Compound **1** was obtained as a transparent viscous solid with a yield of 57%. $^1H$ NMR (300 MHz, $CDCl_3$) $\delta$ 8.33 (d, *J* = 2.2 Hz, 1H), 8.01 (d, *J* = 2.2 Hz, 1H), 7.22 (d, *J* = 3.5 Hz, 1H), 6.39 (d, *J* = 3.5 Hz, 1H), 4.24 (t, *J* = 7.2 Hz, 2H), 1.86-1.82 (m, 2H), 1.31-1.23 (m, 18H); 0.90-0.85 (t, *J* = 3.2 Hz, 3H); $^{13}C$ NMR (75 MHz, $CDCl_3$) $\delta$ 145.8, 143.1, 130.6, 129.3, 122.1, 111.4, 98.8, 44.8, 31.9, 30.3, 29.6, 29.5, 29.5, 29.3, 29.2, 26.8, 22.7, 14.1; FAB MS m/z 365.2 $(M+H)^+$; HRMS (FAB) calculated for $C_{19}H_{30}BrN_2$: 365,1599, found: 365,1592.

### Synthesis of 5-bromo-1-dodecane-1*H*-pyrrole[2,3-*b*]pyridine-3-carboaldehyde. (2)

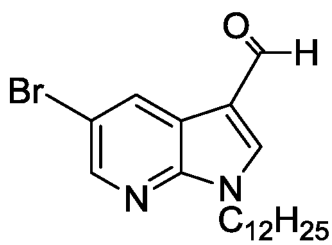

$POCl_3$ (2.37 mL, 24.5 mmol) was added to a solution of 32 mL of dimethylformamide (DMF) in a nitrogen atmosphere at 0 ºC. After stirring for 20 min, 364 mg (1.0 mmol) of 1 dissolved in 8 mL of DMF was added, maintaining the bath at 0 ºC for 30 min. It was then heated at 80ºC for 3h, after which the mixture was cooled at room temperature and the solvent was eliminated at low pressure. The residue was diluted with water and extracted with dichloromethane.

The organic phase was dried with anhydrous $MgSO_4$, it was filtered and the solvent was evaporated in a vacuum. The resulting product was purified in a chromatographic column using silica gel (1:1 hexane: $CH_2Cl_2$, obtaining a yellow solid with a yield of 59%. mp 53-57ºC, $^1H$ NMR (300 MHz, $CDCl_3$) $\delta$ 9.95 (s, 1H), 8.70 (d, *J* = 2.2 Hz, 1H), 8.45 (d, *J* = 2.2 Hz, 1H), 7.87 (s, 1H), 4.31 (d, *J* = 7.2 Hz, 2H), 1.91 (m, 2H), 1.24 (m, 18H), 0.87 (t, *J* = 6.6 Hz, 3H), $^{13}C$ NMR (75 MHz, $CDCl_3$) $\delta$ 184.2, 146.8, 145.8, 138.5, 132.6, 119.0, 115.6, 115.1, 45.7, 31.9, 29.9, 29.6, 29.5, 29.4, 29.3, 29.07, 26.7, 22.7, 14.1; UV-vis ($CH_2Cl_2$, 25 ºC) $\lambda_{max}$ ($\varepsilon$) 264 (26889), 291 (16153); FAB MS m/z 393 $(M+H)^+$; HRMS (FAB) calculated for $C_{20}H_{30}BrN_2O$: 393,1537, found: 393,1541.

**Synthesis of 5-(4-(diphenylamino)phenyl)-1-dodecane-*H*-pyrrole[2,3-b]pyridine-3-carboaldehyde. (3)**

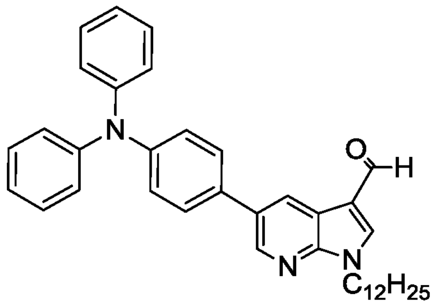

In a sealed tube, 100 mg (0.255 mmol) of **2,** 88.5mg (0.306 mmol) of 4-(diphenylamino)phenylboronic acid and 31 mg (0.027 mmol) of Pd $(PPh_3)_4$ were mixed in 4mL of dry toluene. It was bubbled with a current of $N_2$ for 10 minutes and then 0.3 mL of an aqueous solution of $K_2CO_3$ 2M was added. The reaction was heated at 120ºC for 20 hours in a nitrogen atmosphere, after which it was taken to room temperature and water and dichloromethane were added and it was extracted. The organic phase was dried with anhydrous $MgSO_4$, filtered and the solvent was evaporated at low pressure. The product was purified in a chromatographic column using silica gel (4:1 hexane: Ethyl acetate). Yield 90%, $^1H$ NMR (300 MHz, $CDCl_3$) $\delta$ 10.09 (s, 1H), 8.82 (d, *J* = 2.2 Hz, 1H), 8.74 (d, *J* = 2.2 Hz, 1H), 7.96 (s, 1H), 7.64-7.61 (m, 2H), 7.41-7.35 (m, 4H), 7.29-7.23 (m, 6H); 7.17-7.12 (m, 2H); 4.46 (t, *J* = 7.2 Hz, 2H), 2.0 (m, 2H), 1.35 (m, 18H), 0.97 (t, *J* = 6.6 Hz, 3H), $^{13}C$ NMR (75 MHz, $CDCl_3$) $\delta$ 184.4, 147.6, 147.5, 143.9, 138.3, 132.4,132.3, 129.3, 128.3, 128.2, 124.5, 124.0, 123.1, 117.9, 116.4, 45.7, 31.9, 30.1, 29.6, 29.5, 29.4, 29.3, 29.1, 26.8, 22.7, 14.1; UV-vis ($CH_2Cl_2$, 25 ºC) $\lambda_{max}$ ($\varepsilon$) 300 (108657), 343 (73250); FAB MS m/z 557 ($M^+$); HRMS (FAB) calculated for $C_{38}H_{43}N_3O$: 557,3406, found 557,3396.

**Synthesis of 5-(4-(diphenylamino)phenyl)-1-dodecane-1*H*-pyrrole[2,3-*b*] pyridine-3-(*E*)-2-(E)-2-cyano-but-2-enoic acid (4)**

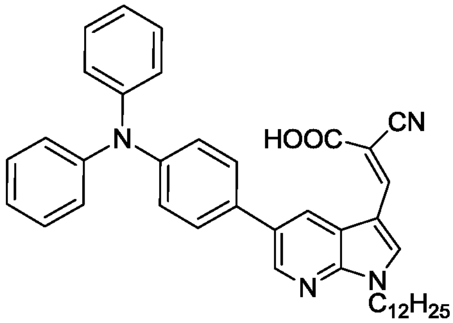

21.6 mg (0.241 mmol) of 2-cyanoacetic acid in 10 mL of acetonitrile and 6 µL of distilled piperidine were added to a two-neck flask with 179 mg (0.313 mmol) of 3 dissolved in 1 mL dichloromethane. The reaction was heated at 90ºC for 24 hours in a nitrogen atmosphere, after which the reaction was cooled at room temperature. The resulting yellow solid was filtered using a filtering crucible and washed with acetonitrile. Yield 49%, mp 255-258 ºC, $^1H$ NMR (300 MHz,

$CDCl_3$) $\delta$ 8.70 (s, 1H), 8.67 (d, $J$ = 2,0 Hz, 1H), 8.59 (s, 1H), 8.30 (d, $J$ = 2.0 Hz, 1H), 7.53-7.50 (m, 2H), 7.29 (m, 4H), 7.22-7.14 (m, 6H); 7.14-7.09 (m, 2H); 4.43 (d, $J$ = 7.38 Hz, 2H), 2.0 (m, 2H), 1.25 (m, 18H), 0.87 (t, $J$ = 6.61 Hz, 3H), UV-vis (CH2Cl2, 25 ºC) $\lambda_{max}$ ($\varepsilon$) 290 (26164), 308 (27338), 379 (25217); MALDI MS m/z 625 $(M+H)^+$; HRMS (MALDI) calculated for $C_{41}H_{44}N_4O_2$: 625,3491, found: 625,3512

## Example 2: optical properties of compounds (3) and (4)

The absorption and emission capacity were measured in a stationary state of compounds (**3**) and (**4**) of the invention. Both had absorption in the UV-vis region. FIG. 1 shows the absorption spectra of the UV-vis of the two molecules studied in $CH_2Cl_2$ ($6.8x10^{-6}$ M for 3 and $2.1x10^{-5}$ M for 4). Observed in the absorption spectra were two absorption bands centered for compound **3** at $\lambda_{max}$=300 and 342 nm and for compound **4** at $\lambda_{max}$=308 and 380 nm, where a displacement of the bands is observed, due to the increase in the conjugation of compound **3** to compound 4.

FIG. 2 shows the emission spectra of **3** and **4** in dichloromethane under the excitation in the maximum absorption peaks $\lambda_{exc}$=352 and 391 nm, respectively. The form of the emission band is modified in the case of compound **4,** in which it is possible to observe the appearance of a 630 nm band and the reduction of the emission at the same concentration with respect to the derivative **3,** maintaining the 470 nm band in common.

This result can be visually observed with the $\lambda$=365 nm lamp, as shown in the sample in FIG. 3, where the flasks used for the study of the emission show the strong fluorescence of compound **3** with respect to compound **4.**

Furthermore, although in a solution compound **3** has greater fluorescence, compound **4** is much more emissive in a solid state. With the aim of researching the behavior of compound **4** in a solid, the absorption and emission spectra were carried out. (FIG.4 and 5) The absorption spectrum has two broad bands of 318 and 378 nm, with a width greater than when in a solution due to the interaction of the molecules in the solid. The emission spectrum of the solid has a single band at 500 nm. ($\lambda_{max}$=391).

This result can be visually observed with the UV-vis lamp at $\lambda$=365 nm, as shown in FIG. 6 (right) where the strong fluorescence of the compound in the solid state may be observed **4.** This result shows that the compounds of the invention are interesting for the application thereof in devices which require a highly fluorescent solid material.

Furthermore, in said compound **4** it is possible to observe the effect of aggregation-induced emission (AIE), since said luminogens are weakly fluorescent in solutions diluted with dichloromethane, yet highly emissive in amorphous powder. In an amorphous state, the molecules aggregate, producing a restriction in the intermolecular vibration, making the aggregate highly emissive, thereby presenting the AIE effect.

The AIE effect of compound **4** has been studied by looking at the absorption and emission in THF and a THF/$H_2O$ solution (FIG.7, 8, 9). FIG 7 shows how adding water produces a change in the UV-vis bands, the 373 nm band of compound **4** practically disappears in 100% of the THF until becoming a shoulder and is displaced up to 359 nm in the mixture of 70%THF/30% $H_2O$ and 40%THF/60%$H_2O$. The band at 310 nm shifts batochromically to 319 nm. When the amount of water is greater, the formation of yellow fluorescent aggregates suspended in the solution (FIG.8) is produced, which shows that the formation of aggregates causes an increase in the fluorescence and said compound is affected by the polarity of the solvent.

In the study of the fluorescence of compound **4** in THF and a THF/$H_2O$ solution in different proportions of water, a change in the emission (FIG.9) is also observed, seeing the appearance of the yellow fluorescent aggregate in a proportion of 10%THF/90%$H_2O$. The emission of said aggregate coincides with the maximum emission of the compound in a solid state at 500 nm.

## Example 3: Electroluminescence performance of compound (3)

To explore the electro-optic behavior of compound 3, single-layer light emitting diodes were prepared which included an active layer of emissive material comprising compound **3** (EM) deposited by spin-coating from a dichloromethane (DCM) solution. It is known that the design of the structure of the device is critical in order to have an energetically favorable charge transfer and high mobility, therefore, the selection of each component that makes up the device is based on the HOMO/LUMO energy values obtained through theoretical calculations. Therefore, **Figure 10A** provides a diagram for the architecture of the single-layer device (device **A**) including the energy levels of the materials. In more detail, device **A** is formed by an ITO anode /PEDOT:PSS (40 nm)/ compound **3** deposited from a DCM solution (300 nm)/Yb metal cathode (150 nm) in which PEDOT:PSS works as a hole injection layer. The current-voltage (IV) curve shown in **Figure 10A** follows the typical characteristics of the diode with a turn-on voltage of ~3V (defined as forward voltage when the current increases exponentially). The electroluminescent (EL) spectrum shown in **Figure 10B** had a single and broad band which becomes more intense under a higher polarization (from 0 to 18 V).

It is worth observing that the form of the EL spectrum does not change when increasing in the driving voltage (**Figure 11A**) and is characterized by the maximum wavelength ($\lambda_{máx}$) at 615 nm and a full-width at half-maximum (FWHM) of ~3200 $cm^{-1}$. The large spectral shift observed in the EL spectrum in comparison with the PL spectrum ($\lambda_{máx}$

= 435 nm with respect to $\lambda_{máx}$ = 600 nm) indicates that the electric-hole recombination mechanism responsible for the EL signal of the device **A** is not the same as in the PL spectrum. A similar mismatch between the PL and EL spectra has been recently observed in 5-(4-nonylphenyl)-7-azaindole (the acceptor moiety of **1**), where the formation of electromers was invoked to explain the EL emission. The photophysical studies of the molecule provided support for this type of behavior, given that a higher concentration of aggregates in the EM layer increased the performance of the OLED.

To do further research on the mechanism involved in the EL, an optimized device architecture with the incorporation of an electron injection layer, 2-(4-*tert*-butylpheny)-5-(4-butylphenyl)-1,3,4-oxadiazole (PBD), was performed. This double-layer device (device **B**) was formed by an ITO anode /PEDOT:PSS (40 nm)/compound **3** from a DCM solution /PBD (20 nm) /Al metal cathode (150 nm), as shown in the box in **Figure 10C.** The IV curve and the turn-on voltage of device **B** slightly differed from that obtained in device **A** (**Figure 10C**). Additionally, **Figure 10D** displays the EL spectrum of the device **B** with an enhancement of a factor of 60 with respect to device **A,** which indicates that the addition of the PBD layer favors the electron-hole recombination in device **B**. In opposition to the EL signal observed in device **A,** the EL spectrum in device **B** is red-shifted when the driving voltage is increased (**Figure 11B**).

To gain further insight as to the origin of the EL signal, a Gaussian deconvolution of the EL spectrum was performed from 8 to 20 V (**Figure 12A**). At lower driving voltages (from 8-10 V) three centered bands at ~437, 555 and 615 nm with their relative contributions (obtained based on the integral intensity) varying from 0.20 to 0.1, 0.75 to 0.51, 0.05 to 0.28, respectively, were required to obtain an accurate fit. On the other hand, at higher voltages (from 13-20 V) only two Gaussian bands centered at ~555 and 615 nm were required. Although the lower-energy component is also present in device **A,** indicating a common origin of these emitting species in both devices, the other two components are only present in the double-layer device (device **B**). One possible explanation for the new components is that they come from the PBD electroluminescence, where $S_1$ and the electromer emission is centered at ~366 and ~ 518 nm, respectively, but these values are far from those found in device **B,** excluding this possibility. On the other hand, several studies have shown that in two-layer devices, the formation of an exciplex or electroplex, meaning, a biomolecular excited complex formed by the electron from the LUMO of the donor to the HOMO of the acceptor molecules via photo or electric field-excitation, at the interfacial area between the organic layers, could contribute to the EL spectra. Taking this fact into account, the exciplex emission ($hv_E$) between compound **1** and PBD is estimated using the following equation:

$$hv_E = E_{D^-/D^+} - E_{A/A} - 0.15\,eV\ (\pm 0.1\,eV),$$

where $E_{D+/D-}$ is the oxidation potential of **3** (0.5 eV) and $E_{A+}/_{A-}$ is the reduction potential of PBD (-2.0 eV). The calculated emission of the exciplex is 2.65 eV (470 nm), which perfectly fits to the 467 nm component and therefore we assign the first component to the PBD exciplex emission. Since the distance to have an exciplex is smaller (0.3-0.4 nm) than the one required to have a electroplex (0.4-0.7 nm), the chance to form an electroplex between PBD and compound **3** is high enough to suggest that the contribution of ~555 nm comes from the electroplex emission. Besides this, the red shift of the electroplex from the exciplex emission can be explained assuming that the electron and hole are much more stabilized than the singlet excited stated of the exciplex, which causes the radiative recombination to appear at lower energy (longer wavelengths) than the $S_1$-exciplex emission.

A detailed analysis of dependence of integral intensity of each component with respect to forward voltage can help us to elucidate the origin of each emissive species. **Figure 12B** shows that the component centered at ~ 615 nm is clearly enhanced at high electric field, indicating that at higher driving voltages more electrons could be directly injected to the LUMO orbital of component **3** and therefore more electromers could be created. In contrast, the other species assigned to exciplexes and electroplexes are reduced when the driving voltage increases, mainly due to the phase separation between the EM layer and PBD layer. Consequently, the red-shift in the EL spectra with respect to the PL emission was attributed to the presence of different types of complexes in the EM layer, which preferentially formed nearest-neighbors hole-electron pairs between the EM and PBD layer. These results highlight the importance of the device structure of an OLED and how small modifications can alter the characteristics of the device.

**Example 4: OLED device fabrication**

For the device fabrication, an indium-tin-oxide (ITO)-coated glass substrate with a sheet resistance of ~109 Ω cm was used as a substrate. Before use, the substrates were first cleaned in an alkaline-detergent water (Hellmanex solution) using an ultrasonic bath for 10 min, followed by sonication for 10 min, consecutively, in water, acetone and isopropanol. After that, the cleaned ITO-glasses (30 min) were subjected to an ultraviolet-ozone treatment to enhance the work function of ITO layer. On the cleaned ITO substrates, a hole injection layer, PEDOT:PSS (Sigma Aldrich, high conductivity), was spin coated at 3000 rpm for 120 seconds and subsequently annealed at 150ºC during 15 minutes. Afterwards, the emissive layer of compound **3,** was spin coated at 1000 rpm for 60 seconds using a solution of 20 mg/ml of **3** in DCM. Next, in the case of double-layer devices, the electron injection layer, 2-(4-*tert*-butylphenyl)-5-(4-biphenylyl)-1,3,4-oxadiazole (PBD, Sigma Aldrich), was spin coated at 5000 rpm for 120 seconds (in cyclohexane) and annealed at 80ºC for 15 minutes. Finally, a 150 nm ytterbium (Yb) or aluminum (Al) electrode was thermally evaporated under a high vacuum on top of the organic layer. Yb and Al were chosen for their low work function and higher stability to oxygen compared to other low work function metals such as alkaline earth metals (Ca, Mg,) used earlier.

**Stationary fluorescence measurements**

The UV-visible steady-state absorption and emission spectra were recorded using a Lamda-950 and Edinburgh fls980 spectrometers, respectively The Edinburgh fls980 spectrometer was corrected for the wavelength dependence of the throughput of the emission monochromator and the sensitivity of the detector. To obtain the photoluminescence spectra of compound **3** in film, the protocol as developed to make the EM layer was followed to obtain proper samples. The different solutions of compound **3** in cyclohexane, toluene, dichloromethane and acetonitrile (5 mg/ml) were spin coated at 1000 rpm for 60 seconds on an ultrasonically cleaned quartz substrate.

**Stationary electroluminescence measurements**

Stationary measurements have been recorded using Edinburgh fls980 fluorimeter (the same equipment used for the stationary fluorescence measurements) where the Xe-lamp (the lamp uses to excite the sample) was blocked in order to only register the electroluminescence spectra.

**IV curves**

All IV curves were measured in the dark at room temperature using a Keihtley 2400 device.

**Example 5: Electroluminescence performance of compound (4)**

The good photophysical properties of compound **4** show the possible use thereof as an active emissive layer in OLED applications. According to the theoretical calculations, where the HOMO-LUMO were found at -5.3 and -2.4 eV, respectively, a double-layer light-emitting device was functional. The inset of **Figure 13A** schematizes the used OLED configuration: ITO anode/PEDOT:PSS (40 nm)/compound **4** from a toluene solution/ (300 nm)/ 2-(4-*tert*-Butylphenyl)-5-(4-biphenylyl)-1,3,4-oxadiazole, (PBD)/ Al metal cathode (150 nm), where the PEDOT:PSS and PBD act as a hole and electron injection layer, respectively. The device current-voltage (IV) curve depicted in Figure 13A shows a typical diode behavior with a turn-on voltage of 4 V.

To get more information about the electroluminescent properties of **4,** the electroluminescence (EL) spectra were recorded. **Figure 13B** shows a very broad EL spectrum spanning from 400 nm to 700 nm, and it becomes more intense under a higher forward polarization. Together with this, as shown in **Figure 14,** upon increasing forward driving voltage, the EL spectra becomes slightly broader (from ~3300 to 3400 $cm^{-1}$), however, in all of them, the maximum wavelength is centered around 520 nm. The EL maximum overlaps nicely with the PL maximum. However, the EL spectra is much broader. A possible explanation could be the presence of additional species around ~460 nm and ~560 nm.

Similar observations report the presence of exciplexes and/or electroplexes in bi-layer devices. A hetero bimolecular complex in the excited state formed in the interface of the layers. The exciplex emission can be estimated using the following equation:

$$h\nu_E = E_{D^-/D^+} - E_{A/A} - 0.15\,eV\ (\pm 0.1\,eV)$$

where $E_{D+}/_{D-}$ is the oxidation potential **4** (0.5 eV) and $E_{A+/A-}$ is the reduction potential of PBD (-2.0 eV). The obtained value of 2.6 eV (470 nm) is in the expected spectral region. The possibility of having an electroplex when an exciplex is observed is fairly high since the distance needed to form an electroplex is smaller (0.4 - 0.7 nm) than the exciplex emission (0.2 - 0.4 nm). It is worth noting that the electroplex emission is significantly red shifted in comparison to the exciplex due to the electron-hole stabilization in HOMO and LUMO of each molecule that form the bimolecular complex. Based on those facts, the red emitting species around 560 nm could be assigned to the electroplex emission.

A similar phenomenon has been observed for a similar molecule using the same double-layer device form compound **3.** In this case, the exciplex and electroplex emission have been observed at the same wavelengths, which support the assumption that the blue and red EL species come mainly from electroplex and exciplexe formation. On the other hand, in the previous study, the emission from the LUMO-HOMO recombination was not observed and instead an electromer emission was observed (homo bimolecular complex formed by identical charged molecules). To comprehend the reason why here the HOMO-LUMO recombination was favored, an analysis of the chemical structures and their implication in the energy diagram should be understood. The dissimilarity between both molecules lies in the electron acceptor group. In the previous case, the azaindole had a connected aldehyde group, now the group consists of a cyano and carboxyl group. The presence of a stronger electron acceptor in the molecule stabilizes the LUMO 0.7 eV, as it has been described in the theoretical calculation, and consequently the possibility of having an electron-hole recombination between two azaindole molecules is reduced (LUMO energy is ~1.2 eV) in favor of the electron-hole recombination in the HOMO-LUMO of the molecule. These results highlight the fact that small modifications in the chemical structure of the emissive molecule could improve optoelectronic properties, as well as allow the color emission of the OLED to be tuned.

Similar to other studies, the current and electroluminescent stability over time in a non-sealed device was tested (**Figure 15**). In this case, the results highlight that even though the current is reduced in a five weeks-aged device, the EL spectra remains constant, which suggest that the degradation on the EM layer is rather small. The differences found on the IV curve could be explained by the degradation of the contacts due to its exposure to oxygen and moisture. Based on that, it is important to highlight that a proper sealing of the device will provide a robust device.

## Claims

1. A compound of formula (I):

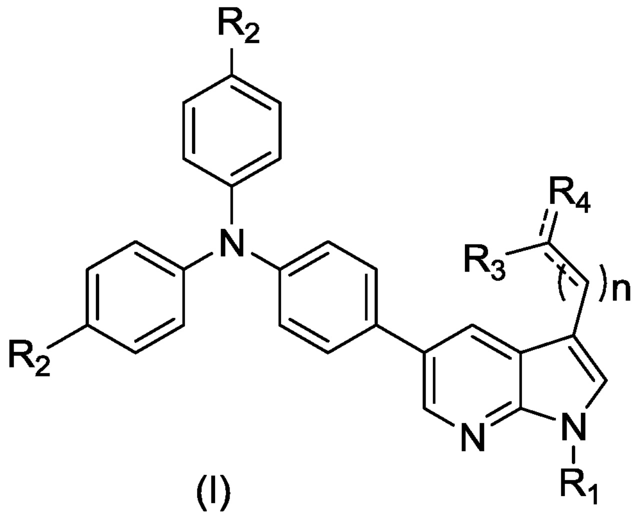

(I)

wherein $R_1$ is a $C_1$-$C_{20}$ alkyl; $R_2$ is selected from H, $C_1$-$C_6$ alkyl or O- $C_1$-$C_6$ alkyl; $R_3$ is selected from H, CN or CHO; $R_4$ is selected from COOH, CN, O or $CONH_2$; n is selected from 0 or 1.
and wherein - - - - represents a bond which may be double or single depending on the rest of the bonds of carbon atoms which make up the bond.

2. The compound according to claim 1, wherein $R_1$ is a $C_4$ to $C_8$ alkyl.

3. The compound according to claim 1, wherein $R_1$ is a $C_{10}$ to $C_{18}$ alkyl.

4. The compound according to the preceding claim, wherein $R_1$ is a $C_{12}$ alkyl.

5. The compound according to any of the preceding claims, wherein $R_2$ is H.

6. The compound according to any of the preceding claims, wherein $R_3$ is H.

7. The compound according to any of the preceding claims, wherein $R_4$ is O.

8. The compound according to any of claims 6 or 7, wherein n is 0.

9. The compound according to any of claims 1, 3, 4, 5 to 8 which has the following formula:

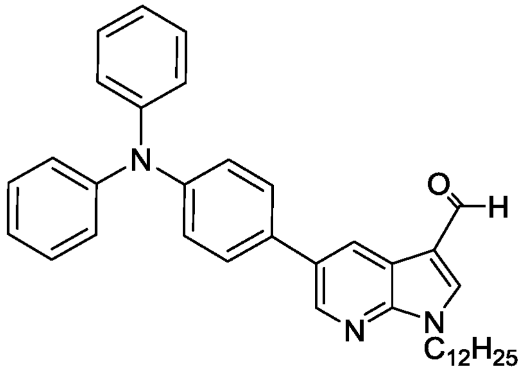

10. The compound according to any of claims 1 to 5, wherein $R_3$ is COOH.

11. The compound according to the preceding claim, wherein $R_4$ is CN.

12. The compound according to any of claims 10 or 11, wherein n is 1.

13. The compound according to any of claims 1, 3, 4, 5 or 10 to 12 which has the following formula:

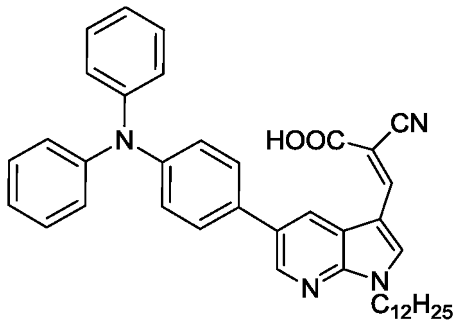

14. A use of a compound of formula (I) according to any of claims 1 to 13 for the fabrication of luminescent or electroluminescent materials.

15. A luminescent or electroluminescent material comprising a compound of formula (I) according to any of claims 1 to 13.

16. A device comprising a luminescent or electroluminescent material according to claim 15.

17. The device according to claim 16, wherein the device is an organic light-emitting diode.

18. The device according to claim 17 comprising: (a) a layer of electroluminescent material and optionally (b) an electron injection layer.

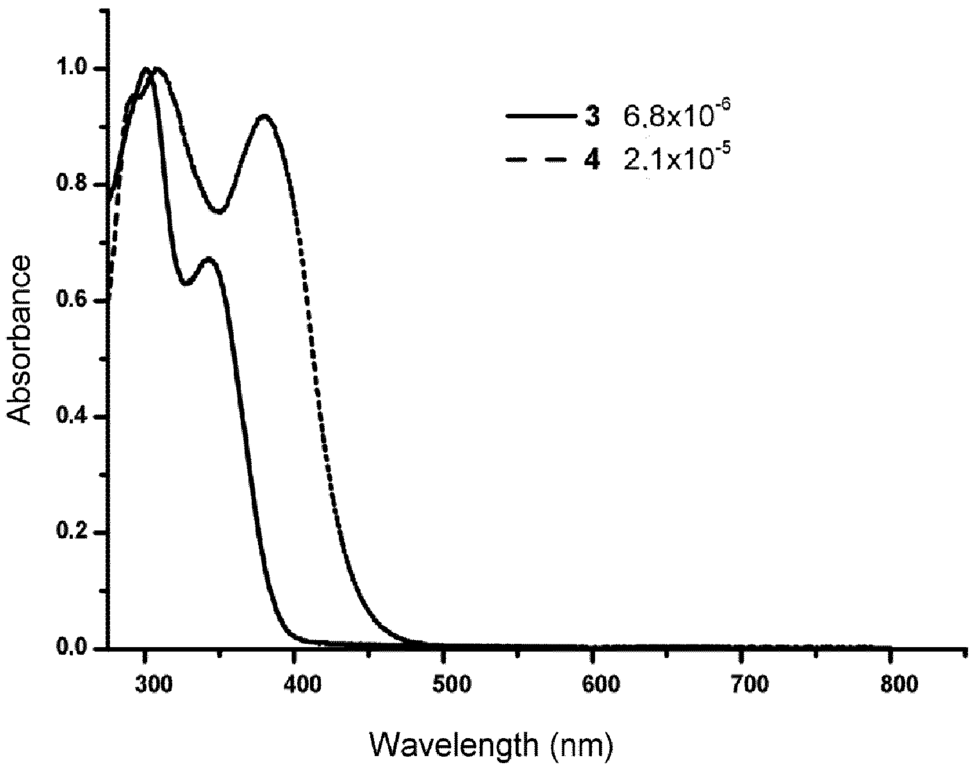
1.0
0.8
0.6
0.4
0.2
0.0
Absorbance
3 6,8x10-6
4 2,1x10-5
300
400
500
600
700
800
Wavelength (nm)

FIG. 1

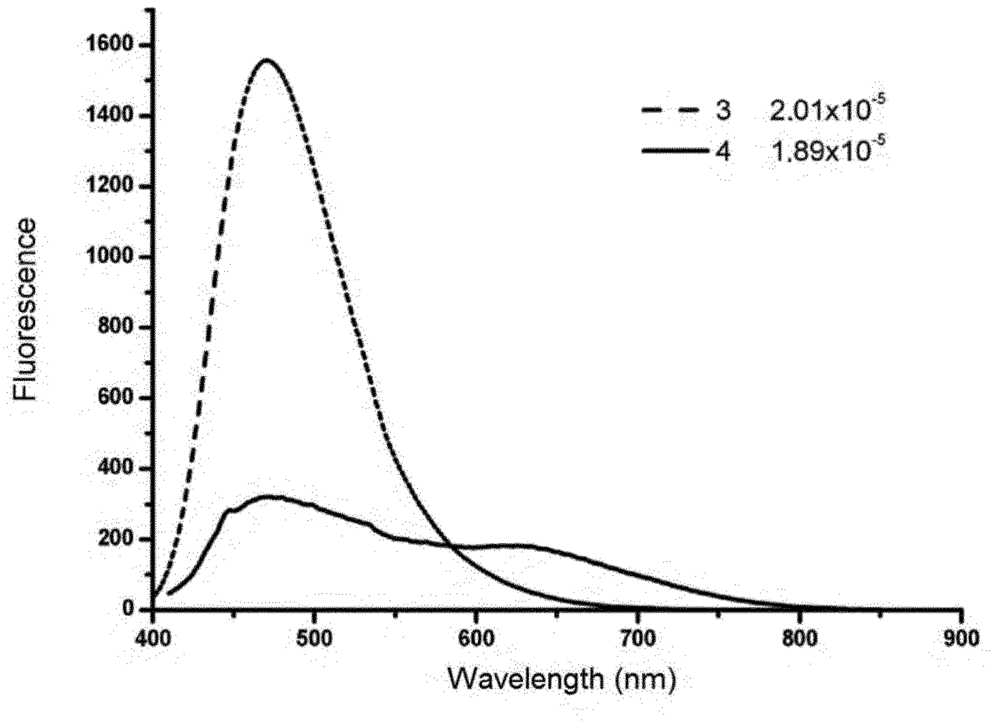
1600
1400
1200
1000
800
600
400
200
0
Fluorescence
3 2.01x10-5
4 1.89x10-5
400
500
600
700
800
900
Wavelength (nm)

FIG. 2

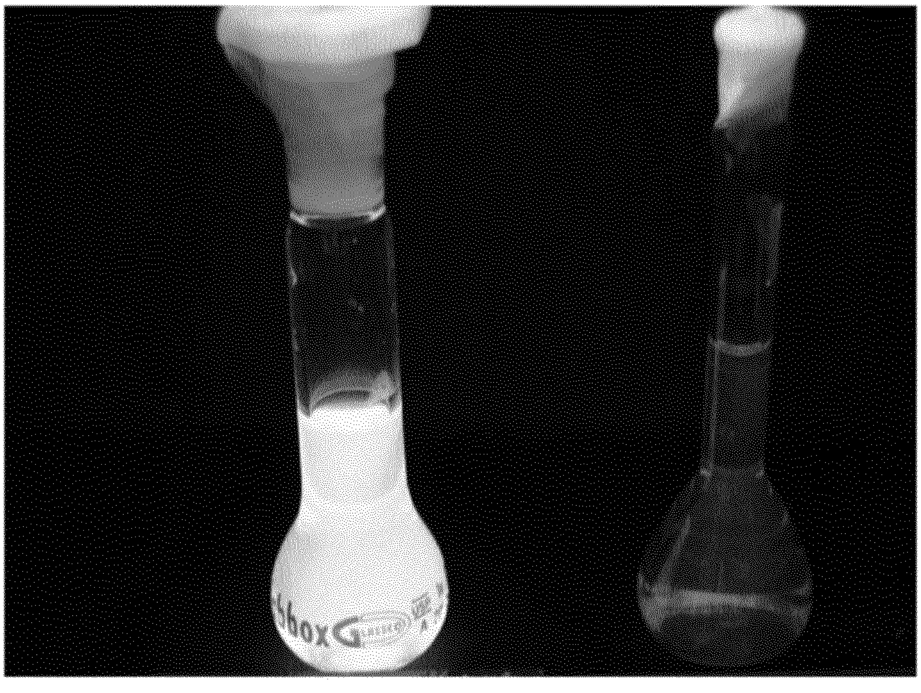

FIG. 3

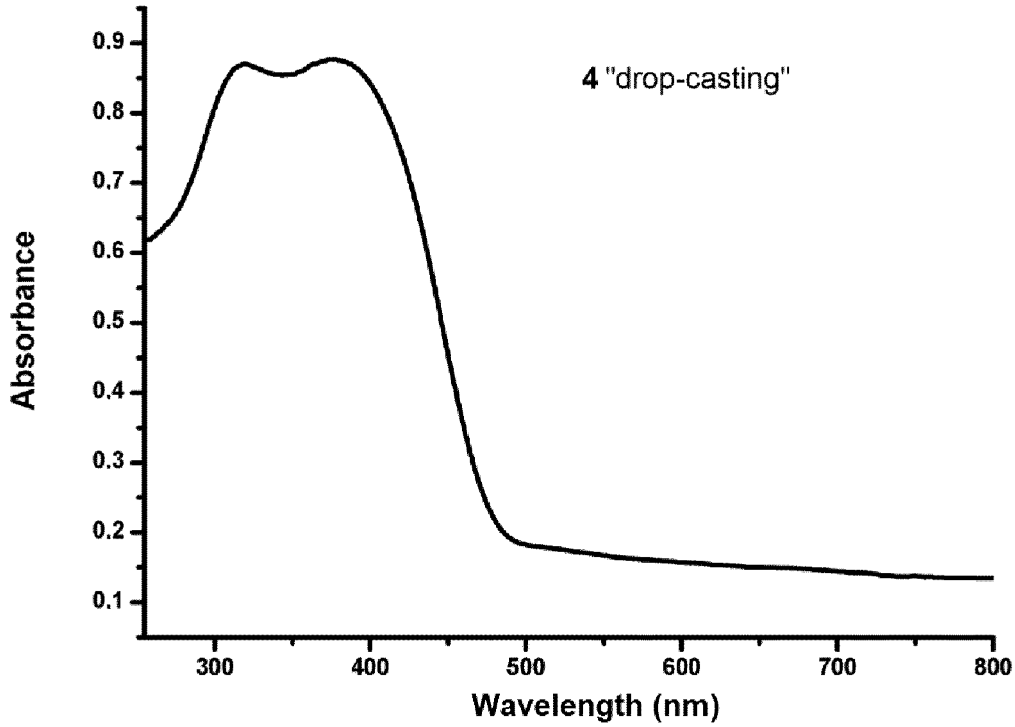
4 "drop-casting"
Absorbance
0.9
0.8
0.7
0.6
0.5
0.4
0.3
0.2
0.1
300
400
500
600
700
800
Wavelength (nm)

FIG. 4

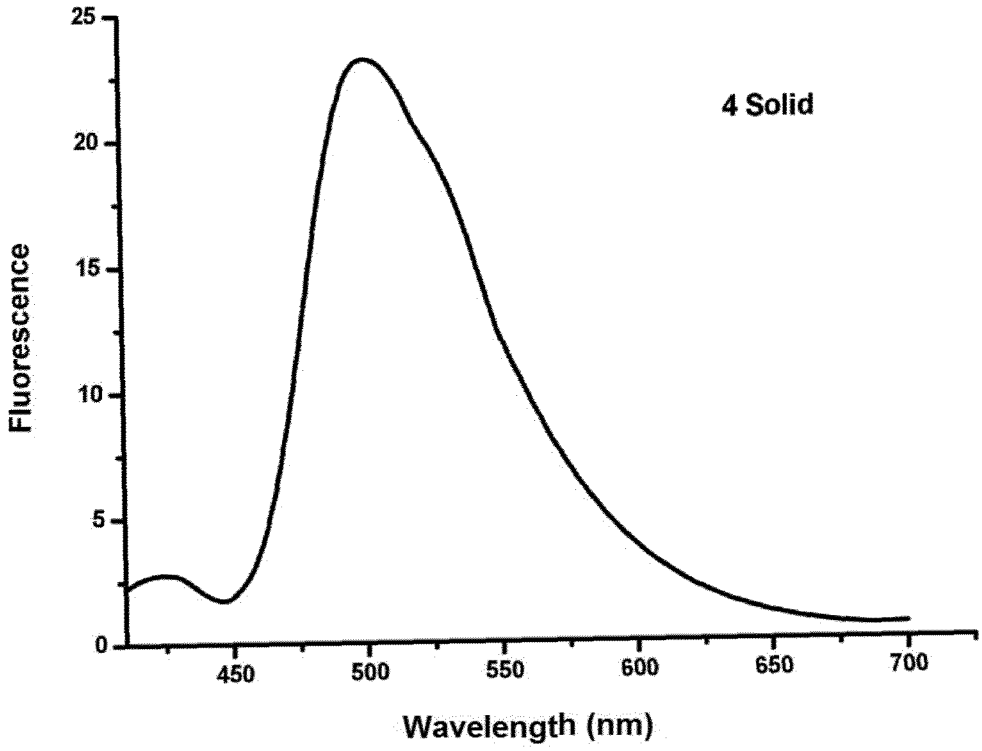
25
20
15
10
5
0
Fluorescence
450
500
550
600
650
700
Wavelength (nm)
4 Solid

FIG. 5

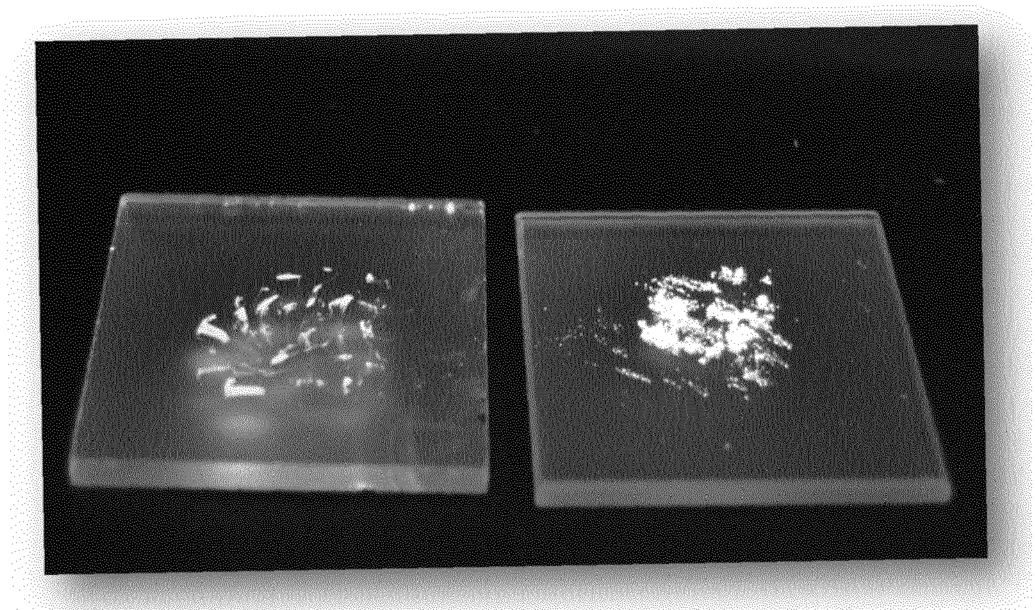

FIG. 6

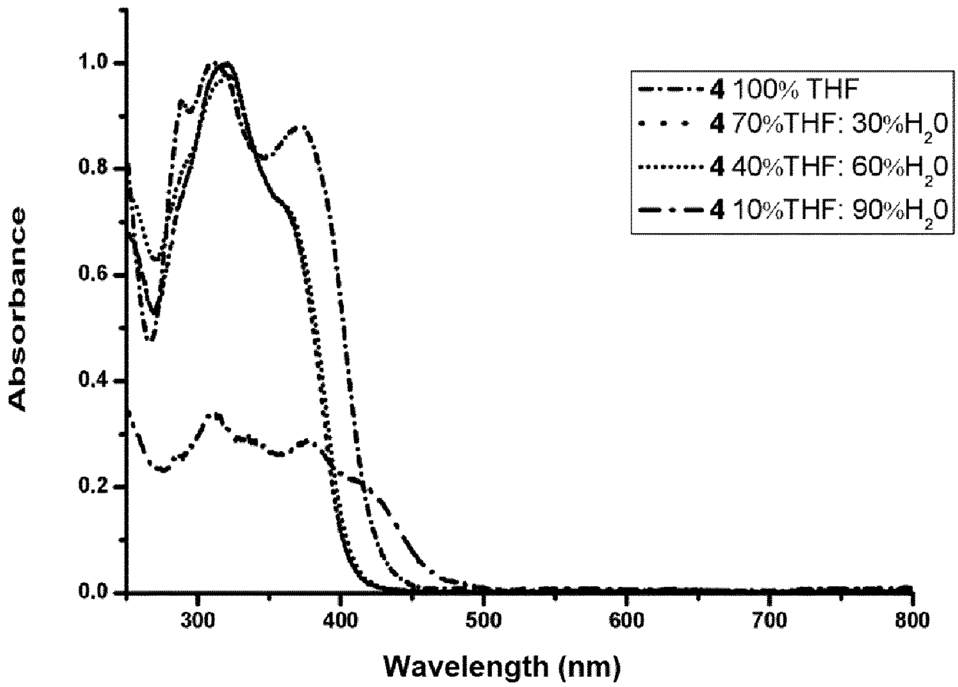

Absorbance
1.0
0.8
0.6
0.4
0.2
0.0
300
400
500
600
700
800
Wavelength (nm)
4 100% THF
4 70%THF: 30%H2O
4 40%THF: 60%H2O
4 10%THF: 90%H2O


FIG. 7

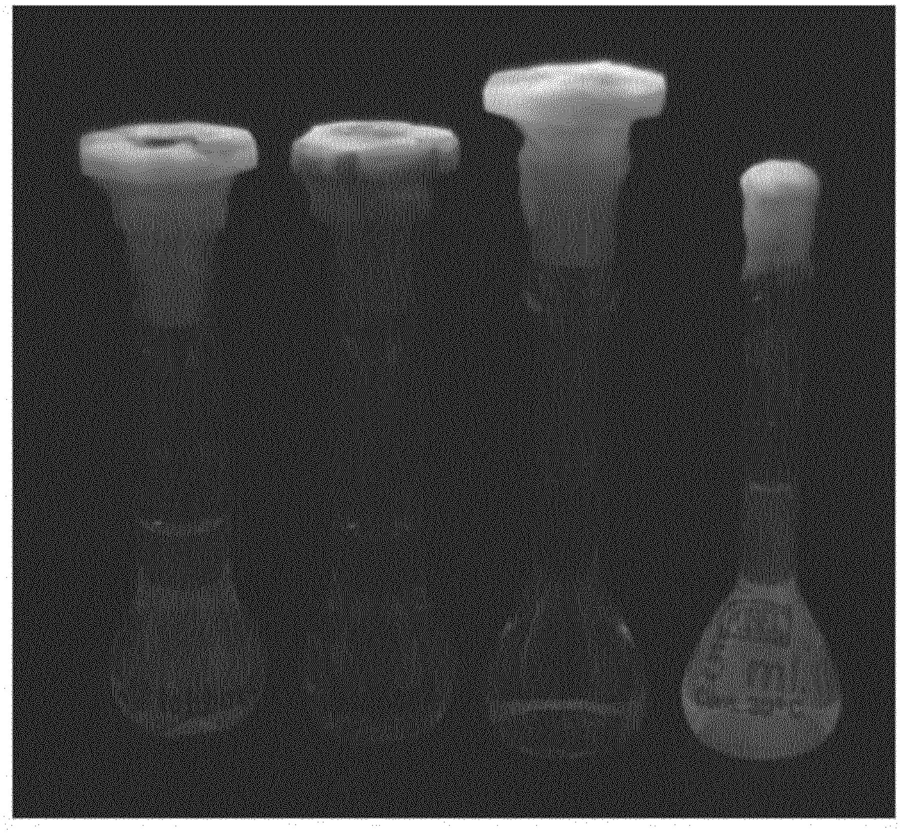

FIG. 8

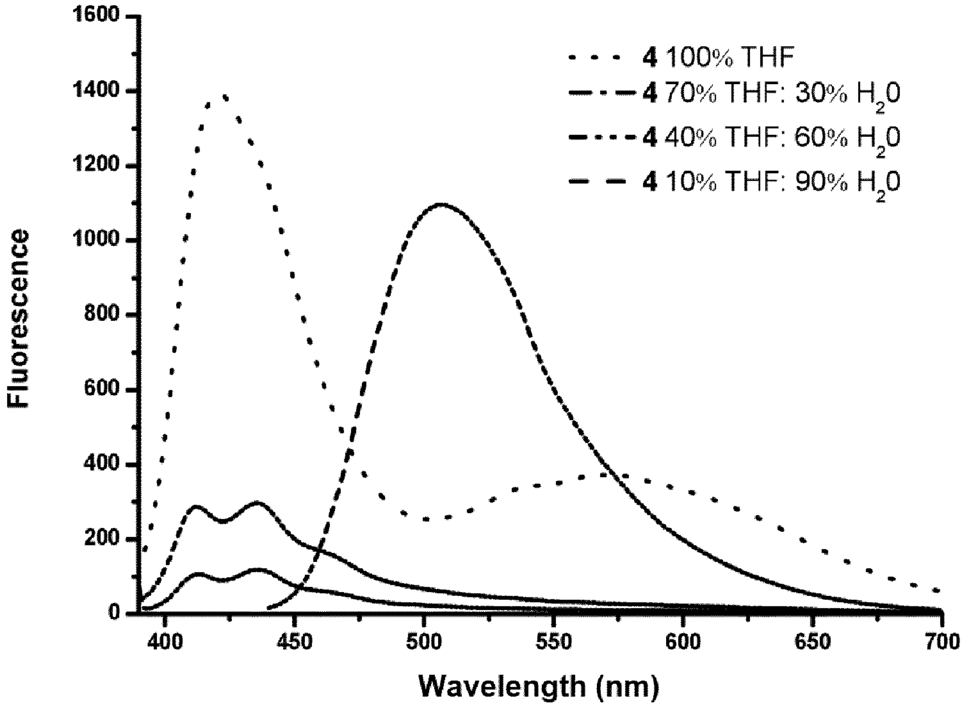

4 100% THF
4 70% THF: 30% H20
4 40% THF: 60% H20
4 10% THF: 90% H20
Fluorescence
Wavelength (nm)
0
200
400
600
800
1000
1200
1400
1600
400
450
500
550
600
650
700


FIG. 9

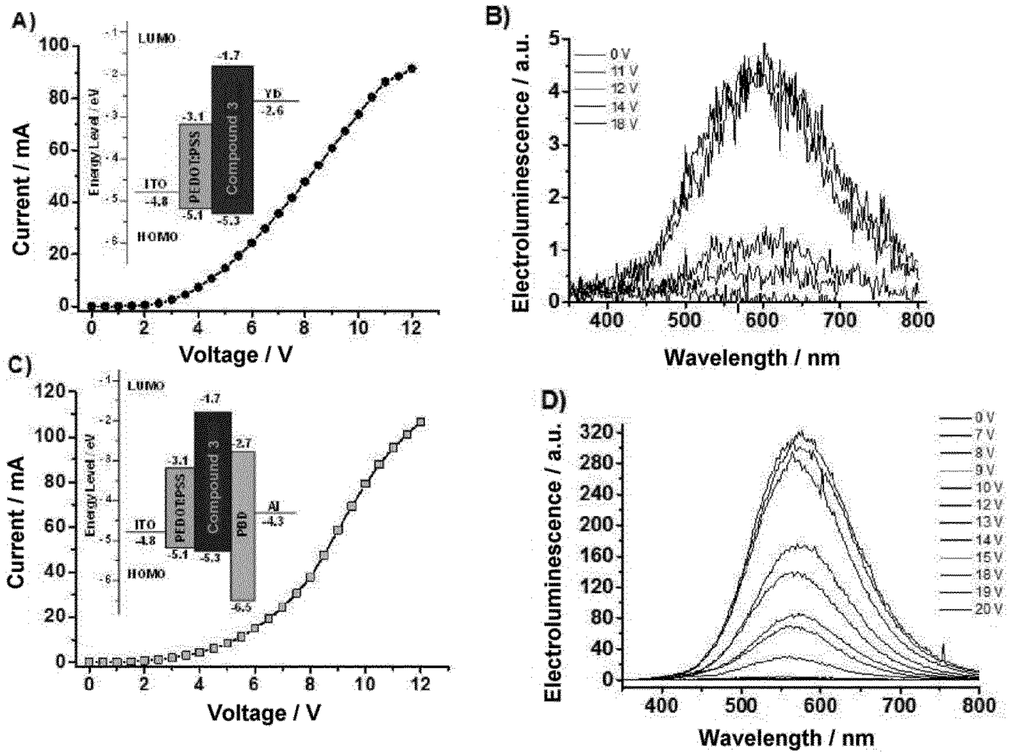
A)
Current / mA
Voltage / V
LUMO
HOMO
Energy Level / eV
ITO
-4.8
PEDOT:PSS
-3.1
-5.1
Compound 3
-1.7
-5.3
Yb
-2.6
B)
Electroluminescence / a.u.
Wavelength / nm
0 V
11 V
12 V
14 V
18 V
C)
Current / mA
Voltage / V
LUMO
HOMO
Energy Level / eV
ITO
-4.8
PEDOT:PSS
-3.1
-5.1
Compound 3
-1.7
-5.3
PBD
-2.7
-6.5
Al
-4.3
D)
Electroluminescence / a.u.
Wavelength / nm
0 V
7 V
8 V
9 V
10 V
12 V
13 V
14 V
15 V
18 V
19 V
20 V

FIG. 10

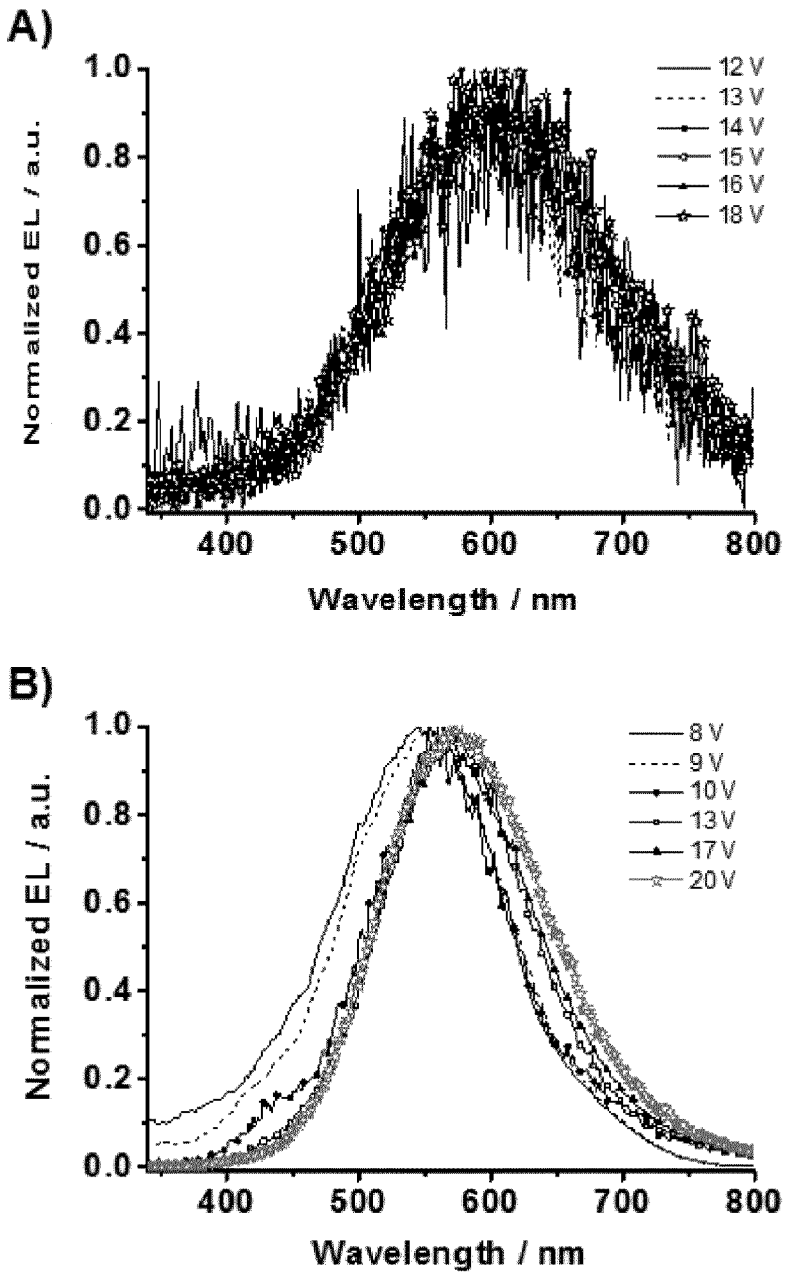
A)
Normalized EL / a.u.
Wavelength / nm
12 V
13 V
14 V
15 V
16 V
18 V
B)
Normalized EL / a.u.
Wavelength / nm
8 V
9 V
10 V
13 V
17 V
20 V

FIG. 11

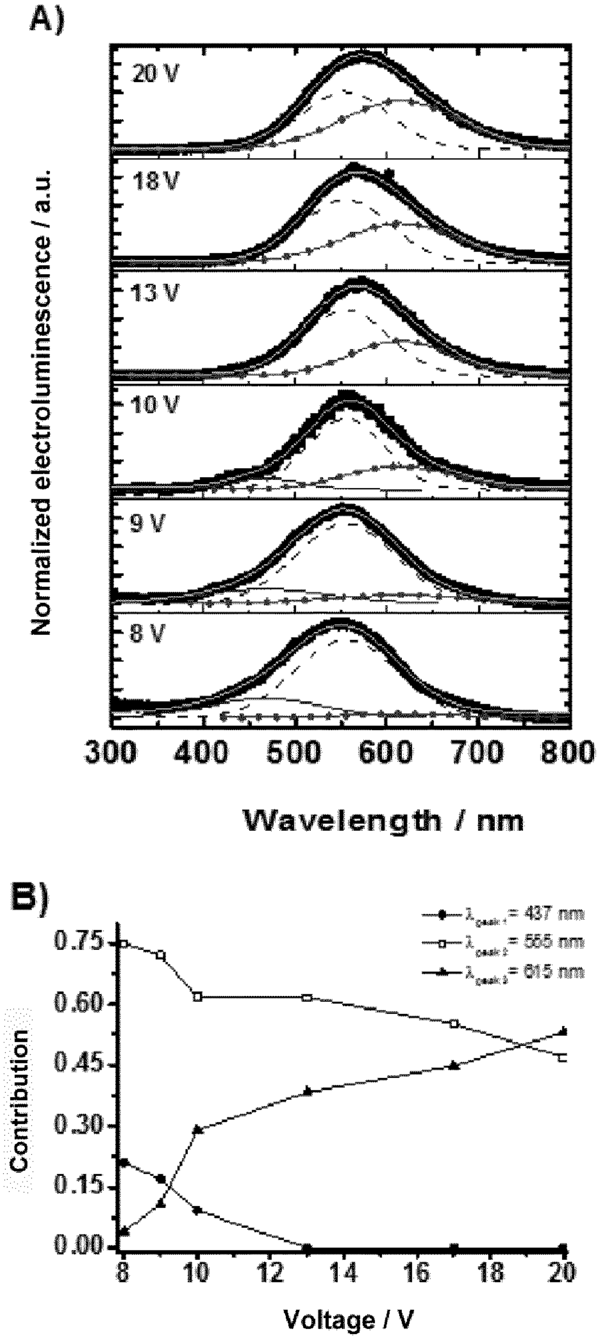
A)
20 V
18 V
13 V
10 V
9 V
8 V
Normalized electroluminescence / a.u.
300
400
500
600
700
800
Wavelength / nm
B)
$\lambda_{peak 1}$ = 437 nm
$\lambda_{peak 2}$ = 555 nm
$\lambda_{peak 3}$ = 615 nm
Contribution
0.75
0.60
0.45
0.30
0.15
0.00
8
10
12
14
16
18
20
Voltage / V

FIG. 12

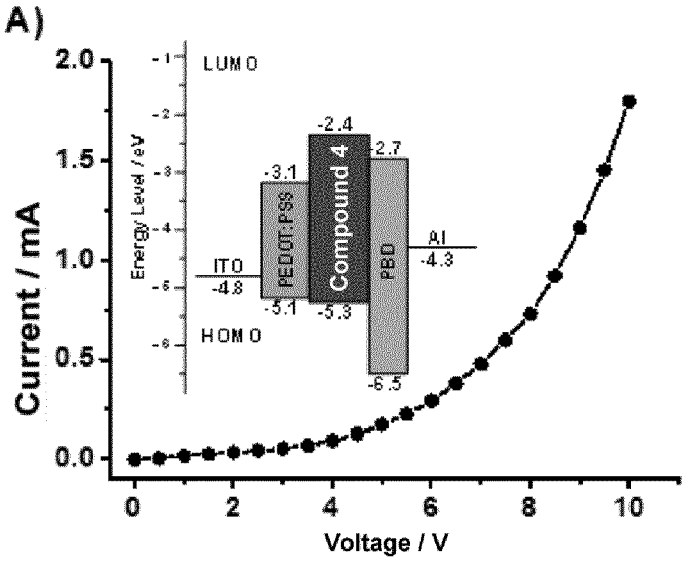
A)
Current / mA
Voltage / V
Energy Level / eV
LUMO
HOMO
ITO
-4.8
PEDOT:PSS
-3.1
-5.1
Compound 4
-2.4
-5.3
PBD
-2.7
-6.5
Al
-4.3

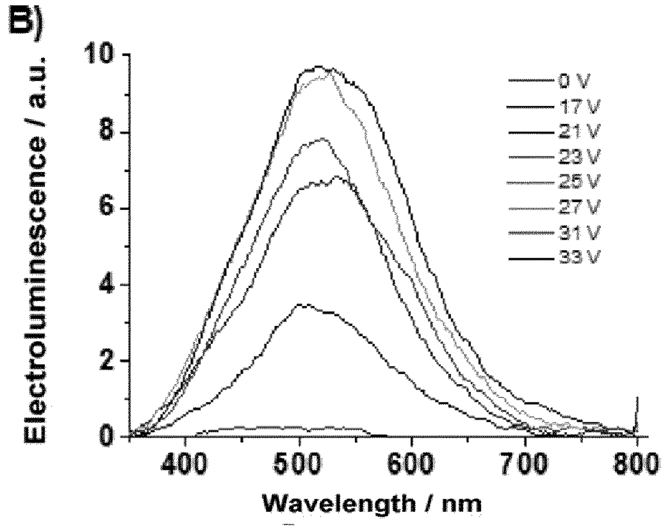
B)
Electroluminescence / a.u.
Wavelength / nm
0 V
17 V
21 V
23 V
25 V
27 V
31 V
33 V

FIG. 13

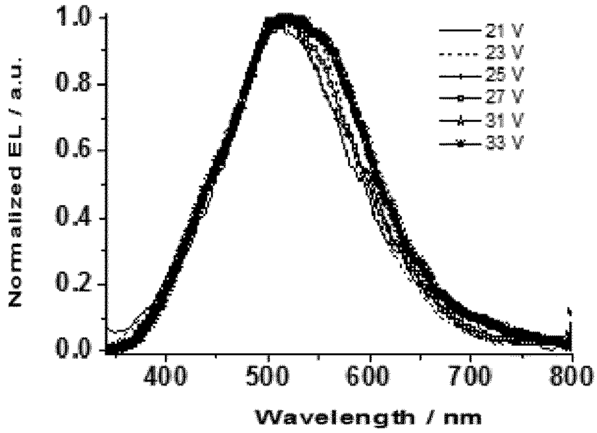
Normalized EL / a.u.
1.0
0.8
0.6
0.4
0.2
0.0
400
500
600
700
800
Wavelength / nm
21 V
23 V
25 V
27 V
31 V
33 V

FIG. 14

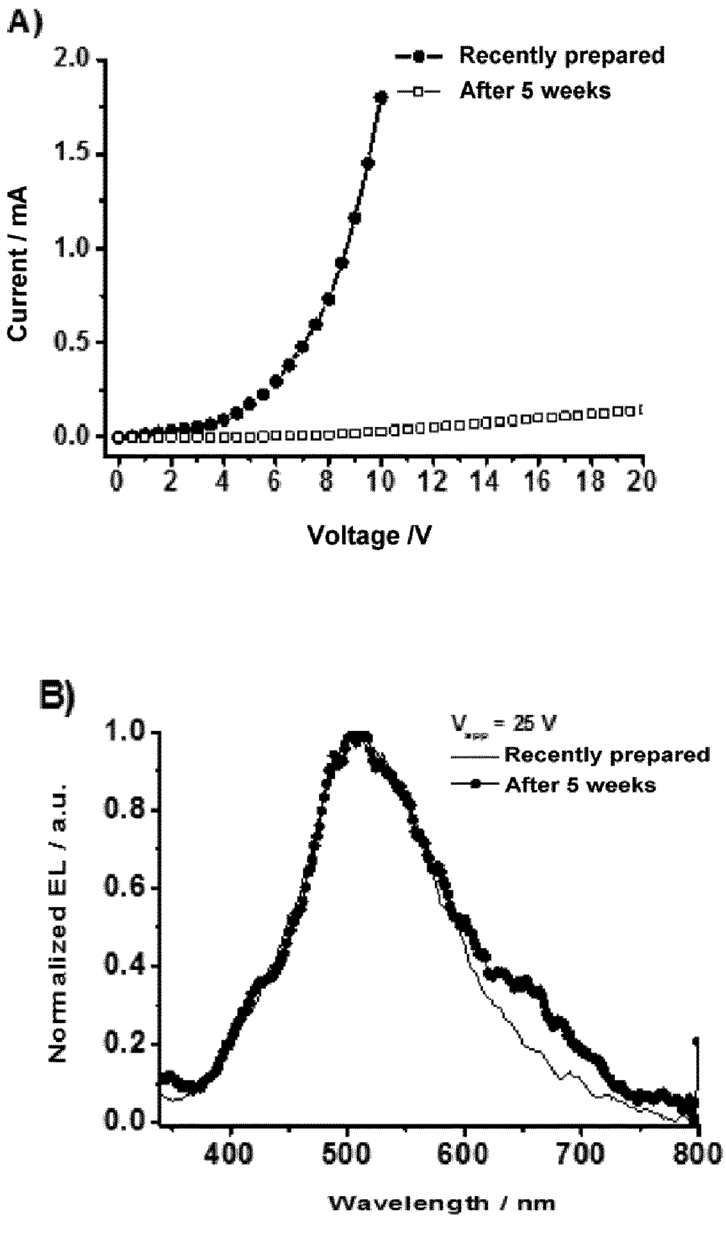
A)
Recently prepared
After 5 weeks
Current / mA
Voltage /V
2.0
1.5
1.0
0.5
0.0
0
2
4
6
8
10
12
14
16
18
20
B)
$V_{app}$ = 25 V
Recently prepared
After 5 weeks
Normalized EL / a.u.
Wavelength / nm
1.0
0.8
0.6
0.4
0.2
0.0
400
500
600
700
800

FIG. 15

# INTERNATIONAL SEARCH REPORT

International application No
PCT/ES2017/070351

**A. CLASSIFICATION OF SUBJECT MATTER**
INV. C07D471/04 A23G1/48 A23G1/50 A23G1/54 A23P10/10
A23P20/10 A23G3/56 A23L19/00
ADD.

According to International Patent Classification (IPC) or to both national classification and IPC

**B. FIELDS SEARCHED**

Minimum documentation searched (classification system followed by classification symbols)
C07D A23G A23P A23L

Documentation searched other than minimum documentation to the extent that such documents are included in the fields searched

Electronic data base consulted during the international search (name of data base and, where practicable, search terms used)

EPO-Internal, CHEM ABS Data, WPI Data

**C. DOCUMENTS CONSIDERED TO BE RELEVANT**

| Category* | Citation of document, with indication, where appropriate, of the relevant passages | Relevant to claim No. |
|---|---|---|
| A | SHU-BIN ZHAO, SUNING WANG: "Luminescence and reactivity of 7-azaindole derivatives and complexes", CHEMICAL SOCIETY REVIEWS, vol. 39, no. 8, 24 Junio 2010 (2010-06-24), páginas 3142-3156, XP002774080, DOI: 10.1039/C001897J páginas 3143-3150, punto 3. "Luminescent 7-azaindolyl complexes" y el punto 4. "Luminescent 7-azaindole derivatives and their complexes";páginas 3154-3155, "Conclusiones " ----- -/-- | 1-18 |

[X] Further documents are listed in the continuation of Box C. [ ] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 27 September 2017 | 13/10/2017 |

| Name and mailing address of the ISA/ | Authorized officer |
|---|---|
| European Patent Office, P.B. 5818 Patentlaan 2<br>NL - 2280 HV Rijswijk<br>Tel. (+31-70) 340-2040,<br>Fax: (+31-70) 340-3016 | Sen, Alina |

2

Form PCT/ISA/210 (second sheet) (April 2005)

page 1 of 2

**INTERNATIONAL SEARCH REPORT**

International application No
PCT/ES2017/070351

C(Continuation). DOCUMENTS CONSIDERED TO BE RELEVANT

| Category* | Citation of document, with indication, where appropriate, of the relevant passages | Relevant to claim No. |
|---|---|---|
| A | CHUN LIU, XIAOFENG RAO, XINLONG SONG, JIESHAN QIUA, ZILIN JINA: "Palladium-catalyzed ligand-free and aqueous Suzuki reaction for the construction of (hetero)aryl-substituted triphenylamine derivatives", RSC ADVANCES, vol. 3, 2013, páginas 526-531, XP002774081, DOI: 10.1039/c2ra22275b página 526, Esquema 1; página 528, Tabla 3; páginas 529-530, Tabla 4 ----- | 1-18 |
| A | YIRANG IM ET AL: "Above 20% External Quantum Efficiency in Thermally Activated Delayed Fluorescence Device Using Furodipyridine-Type Host Materials", CHEMISTRY OF MATERIALS, vol. 26, no. 3, 11 Febrero 2014 (2014-02-11), páginas 1413-1419, XP055213977, ISSN: 0897-4756, DOI: 10.1021/cm403358h página 1414, Esquema 1, compuesto 3TPAPFP y 4TPAPFP; página 1415, Figura 1; página 1416, Figuras 2 and 3; página 1417, Figuras 6 y 7; página 1419, Figura 10 y " Conclusiones " ----- | 1-18 |
| A | MENG LIAN, YUE YU, JIANG ZHAO, ZUAN HUANG, XIAOLONG YANG, GUIJIANG ZHOU, ZHAOXIN WU, DONGDONG WANG: "Novel phosphorescent polymers containing both ambipolar segments and functionalized IrIII phosphorescent moieties: synthesis, photophysical, redox, and electrophosphorescence investigation", JOURNAL OF MATERIALS CHEMISTRY C, no. 2, 2014, páginas 9523-9535, XP002774082, DOI: 10.1039/C4TC01626B pages 9524-9525, "Materials and synthesis", véase L1 y L2; página 9527, compuesto Tz-Am y Py-Am ----- | 1-18 |
| A | MI TIAN, CHAO WANG, LIGONG WANG, KAI LUO, AN ZHAO, CANCHENG GUO: "Study on the synthesis and structure-effect relationship of multi-aryl imidazoles with their fluorescence properties", JOURNAL OF BIOLUMINESCENCE AND CHEMILUMINESCENCE, vol. 29, no. 5, 2014, páginas 540-548, XP002774083, DOI: 10.1002/bio.2580 página 541, Figura 1, compuesto 6; página 544, Figura 5, compound 6; página 547, Figura 9 ----- | 1-18 |

2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- ES 2016070094 W
- ES 201530966


### Non-patent literature cited in the description


- *Chem. Soc. Rev.,* 2012, vol. 41, 211
- *J. Mater. Chem. C,* 2014, vol. 2, 3756
- *Chem. Soc. Rev.,* 2014, vol. 43, 4494
- *Chem. Rev.,* 2015, vol. 115, 11718
- *Soft Matter,* 2013, vol. 9, 4564